(19) Europäisches Patentamt
European Patent Office
Office européen des brevets

(11) **EP 3 647 406 B1**

(12) **FASCICULE DE BREVET EUROPEEN**

(45) Date de publication et mention
de la délivrance du brevet:
**23.06.2021 Bulletin 2021/25**

(51) Int Cl.:
**C12M 1/00** *(2006.01)*  **C12M 1/34** *(2006.01)*
**C12M 1/09** *(2006.01)*

(21) Numéro de dépôt: **19204359.4**

(22) Date de dépôt: **21.10.2019**

(54) **DISPOSITIF DE CAPTURE, DE TRANSPORT ET DE DIFFUSION DE LUMIÈRE POUR UN PHOTO-BIOREACTEUR**

VORRICHTUNG ZUR AUFNAHME, ZUM TRANSPORT UND ZUR DIFFUSION VON LICHT FÜR EINEN FOTO-BIOREAKTOR

DEVICE FOR CAPTURE, TRANSPORT AND LIGHT DIFFUSION FOR A PHOTOBIOREACTOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.10.2018 FR 1801155**
**24.04.2019 FR 1904326**

(43) Date de publication de la demande:
**06.05.2020 Bulletin 2020/19**

(73) Titulaire: **Nénuphar**
**66370 Pézilla la Rivière (FR)**

(72) Inventeur: **DUONG, Frédéric**
**66370 Pézilla La Rivière (FR)**

(74) Mandataire: **Delaveau, Sophie**
**Lambert & Associés**
**18 avenue de l'Opéra**
**75001 Paris (FR)**

(56) Documents cités:
**EP-A1- 0 999 265     WO-A1-2016/087779**

**Description**

La présente invention concerne un dispositif de capture, de transport et de distribution de lumière pour un photo-bioréacteur, en particulier pour équiper des bassins de production de microalgues.

Le développement de la culture algale, en particulier micro-algale en vue d'obtenir un biocarburant photosynthétique, des aliments humains ou animaux ou des composés nutraceutiques issus de la biomasse algale, ne pourra être assuré à grande échelle qu'en réduisant significativement le coût de construction des photo-bioréacteurs et de leur exploitation. En outre, la production algale n'est pas réservée aux seules régions chaudes et ensoleillées, et il faut envisager des solutions techniques permettant d'augmenter la production algale dans des pays situés aux latitudes des régions tempérées, tout en minimisant les besoins en chaleur nécessaire au maintien à la température de croissance adaptée selon les souches de micro algues.

On connaît déjà des photo-bioréacteurs qui équipent des bassins en circuit fermé, tel que celui décrit dans le document WO 2013/011448. Il comprend un bassin fermé par un support de manches en matériau souple remplies de liquide pour capter et transmettre de la lumière au travers de la solution algale contenue dans le bassin.

Si ce bassin a permis de résoudre de nombreux problèmes techniques et économiques tels que l'augmentation de la productivité du bassin et la réduction des coûts de fabrication des manches qui diffusent la lumière, il nécessite encore un équipement relativement conséquent, une fabrication de composants coûteuse et complexe à mettre en œuvre. En outre, cette technologie fermée ne s'applique qu'à des petits bassins pour la production de spiruline, de sorte qu'elle ne permet pas une production d'algues à grande échelle.

En outre, la concentration de lumière vers les manches est complexe, car elle fait appel à des lentilles mobiles qui doivent être dirigées dynamiquement et précisément vers les manches.

Par ailleurs, la surface d'exposition à la lumière est restreinte à la surface des orifices du support dans lesquels débouchent les manches, le reste du support occultant l'intégralité de la surface libre du bassin.

Cette technologie souffre également d'un problème d'encrassement externe des manches par le développement d'un biofilm au contact entre les manches et la solution algale. Il est donc nécessaire de vider régulièrement les manches puis de les retirer de la solution afin de les nettoyer WO2016087779 divulgue un photobioréacteur avec au moins un distributeur de lumière flottant et de forme tubulaire, cylindrique, s'étendant longitudinalement selon un axe X, dont les dimensions sont adaptées aux dimensions du photo-bioréacteur.

La présente invention vise donc à proposer une solution aux problèmes précédents et, en particulier à augmenter la production spécifique de micro algues en optimisant la distribution de la lumière, en améliorant les principaux paramètres photosynthétiques (température, photo inhibition, circulation), et en limitant l'évaporation d'eau du bassin.

L'invention vise également à proposer une solution simple et rapide à mettre en œuvre, autonome, économique et polyvalente, ceci quel que soit la taille et la profondeur du bassin, c'est-à-dire à la fois facilement et rapidement évolutive en fonction des conditions et ayant un champ d'application très large, qui va des petits bassins existants à des bassins dits « open ponds » de grandes dimensions, en étang et en mer, pour une production extensive de grande ampleur.

À cette fin, l'invention a pour objet un dispositif de capture, de transport et de diffusion de lumière dans une solution contenue dans un réservoir, le dispositif comprenant une manche cylindrique, en matériau transparent à la lumière transportée et diffusée, la manche étant destinée à être remplie d'un liquide propre à transmettre et diffuser la lumière et à équilibrer la pression extérieure pour maintenir la manche sous forme cylindrique. Selon l'invention, le dispositif comprend, en outre, une cloche étanche, transparente à la lumière captée, transportée et diffusée, et formée au moins en partie par deux parois séparées par un interstice étanche (e) rempli en tout ou en partie par du gaz, la cloche comprenant une partie en dôme prolongée par une partie tubulaire, une première extrémité de la manche étant destinée à être bloquée contre la cloche de sorte qu'en position d'utilisation, la cloche flotte librement dans la solution et maintient au moins la partie en dôme émergée et la manche remplie de liquide immergée et étendue verticalement sous la cloche en adoptant une forme cylindrique.

Le terme "transparent" utilisé dans la description et les revendications désigne un matériau qui laisse passer la lumière, et ce terme doit être compris au sens large englobant un matériau translucide. Ce qui importe c'est que la lumière soit captée et canalisée par le dispositif pour être transmise à la solution algale.

L'invention permet ainsi d'adresser simultanément tous les problèmes précédents, notamment celui de l'augmentation de la production d'algue en améliorant la transmission de lumière et les principaux paramètres photosynthétiques tout en assurant une bonne isolation thermique et une circulation favorisée par le mouvement des manches dans le bassin, et celui de la réduction de l'évaporation de l'eau des bassins sans réduire la surface de solution exposée à la lumière du soleil.

En particulier, l'invention permet un entretien naturel et automatique de la surface immergée des dispositifs qui limite la formation du biofilm.

En outre, la solution proposée est simple et rapide à mettre en œuvre, autonome, économique et polyvalente.

Selon d'autres modes de réalisation, qui peuvent être combinés entre eux :

- la partie en dôme de la cloche peut être hémisphérique ;
- la partie en dôme de la cloche peut être à double paroi ;
- la partie en dôme de la cloche peut être à simple paroi et la partie tubulaire être à double paroi ;
- l'interstice étanche (e) séparant les doubles parois peut être rempli exclusivement de gaz ;
- l'interstice étanche (e) séparant les doubles parois peut être rempli d'une mousse expansée à cellules fermées remplies de gaz, telle qu'une mousse en polyurethane ;
- la partie en dôme peut comprendre un évent tubulaire traversant la partie en dôme de manière étanche et comprenant une valve de maintien de pression atmosphérique équipée d'une membrane souple étanche qui peut se déformer sous la pression atmosphérique ;
- la partie tubulaire de la cloche peut comprendre une gorge annulaire destinée à recevoir, en utilisation, une bague torique extensible de maintien étanche d'une première extrémité de fixation de la manche contre la partie tubulaire ;
- la partie tubulaire de la cloche peut présenter une surface conique se rétrécissant vers la partie en dôme ;
- la cloche peut comprendre une première pièce comprenant la partie en dôme prolongée par une portion cylindrique, et une seconde pièce comprenant une portion cylindrique munie d'un col destiné à être en contact périphérique étanche avec une surface intérieure de la première pièce, en position d'utilisation, afin de délimiter un interstice étanche, en utilisation, entre la portion cylindrique de la première pièce et la portion cylindrique de la seconde pièce ;
- la cloche peut comprendre une première pièce comprenant la partie en dôme prolongée par une portion cylindrique, et une seconde pièce comprenant une portion cylindrique munie d'un épaulement destiné à être en contact périphérique étanche avec la portion cylindrique de la première pièce, en position d'utilisation, la seconde pièce étant constituée par un anneau en un matériau à cellules fermées remplies de gaz ;
- la portion cylindrique de la deuxième pièce peut présenter une surface réfléchissante pour réfléchir, en utilisation, la lumière provenant de la partie en dôme vers la manche ;
- la manche peut comprendre une seconde extrémité munie d'une fermeture étanche lestée ;
- une extrémité inférieure de la manche peut comprendre des cellules photovoltaïques dirigées, en utilisation, vers la cloche, et reliées à un accumulateur relié à des diodes électroluminescentes dirigées, en utilisation, vers la cloche, l'accumulateur étant programmé pour accumuler de l'électricité le jour et pour alimenter les diodes luminescentes la nuit ou pendant des périodes de faible intensité solaire ;
- l'accumulateur peut être relié à un résonateur récepteur de puissance capable de transformer en électricité l'énergie magnétique transmise, en position d'utilisation, par des résonateurs émetteurs de puissance équipant le fond du réservoir ;
- l'extrémité inférieure de la manche peut comprendre un réflecteur conformé pour réfléchir la lumière vers la manche ;
- le réflecteur peut être convexe ou concave ;
- le réflecteur peut présenter une surface de contact avec l'extrémité inférieure de la manche inférieure à une surface de ladite extrémité inférieure de la manche de manière à laisser passer une partie de la lumière au travers de l'extrémité inférieure de la manche ;
- le réflecteur peut être ajouré de manière à laisser passer une partie de la lumière vers l'extrémité inférieure de la manche ;
- la cloche peut être en un matériau choisi parmi le polypropylène (PP), le poly(méthacrylate de méthyle) (PMMA), le poly(téréphtalate d'éthylène) (PET), et un plastique transparent traité anti UV ;
- la manche peut être en un matériau choisi parmi le polyester, le polypropylène (PP) et tout autre matériau transparent en film, d'épaisseur de quelques dizaines de micromètres ; et/ou
- la manche, en utilisation, peut être remplie d'eau salée et préalablement dégazée.

L'invention a également pour objet un photo-bioréacteur pour la production d'une solution algale concentrée comportant un réservoir contenant la solution algale, et au moins un dispositif de capture, de transport et de diffusion de lumière précédent, en flottage libre à la surface de la solution algale.

Selon d'autres modes de réalisation, qui peuvent être combinés entre eux :

- le photo-bioréacteur peut comprendre, en outre, des moyens d'agitation de la solution algale de capables de générer un courant apte à déplacer les dispositifs de transport et de diffusion de lumière précédent en flottage libre à la surface de la solution ;
- les moyens d'agitation peuvent comprendre un distributeur d'un gaz sous pression, en particulier du dioxyde de carbone ;
- le photo-bioréacteur peut comprendre, en outre, une structure maillée dont chaque maille est de dimension strictement supérieure à un diamètre maximal de la cloche dudit au moins un dispositif de transport et de diffusion de lumière, de manière à permettre un mouvement aléatoire délimité d'un dispositif de transport et de diffusion de lumière compris dans une maille ;

- la structure maillée peut être constituée par un filet souple et flottant ; et/ou
- le photo-bioréacteur peut comprendre, en outre, des résonateurs émetteurs de puissance équipant le fond du réservoir.

L'invention a également pour objet un dispositif de nettoyage d'un dispositif de capture, de transport et de diffusion de lumière précédent, le dispositif de nettoyage comprenant un support circulaire munie d'au moins un galet motorisé rotatif pour l'entraînement en rotation de la cloche du dispositif de capture, de transport et de diffusion de lumière en position de nettoyage, le support comprenant, en outre, au moins une brosse agencée sous le support et destinée à être en contact avec la manche du dispositif de capture, de transport et de diffusion de lumière en position de nettoyage.

Avantageusement, le support peut comprendre, en outre, des flotteurs.

L'invention a également pour objet un photo-bioréacteur précédent, comprenant, en outre, au moins un dispositif de nettoyage précédent.

D'autres caractéristiques de l'invention seront énoncées dans la description détaillée ci-après, faite en référence aux dessins annexés, qui représentent, respectivement :

[Fig. 1], une vue schématique en coupe d'un dispositif de capture, de transport et de diffusion de lumière selon l'invention en position d'utilisation dans un réservoir ;

[Fig. 2], une vue schématique partielle en coupe du dispositif de la figure 1 muni d'une valve d'équilibrage hydrostatique ;

[Fig. 3], une vue schématique en coupe d'un dispositif de transport et de diffusion de lumière selon l'invention, en cours de remplissage ;

[Fig. 4], une vue schématique en coupe d'un premier mode de réalisation alternatif d'une cloche du dispositif de capture de transport et de diffusion de lumière selon l'invention ;

[Fig. 5], une vue schématique en coupe d'un deuxième mode de réalisation alternatif d'une cloche du dispositif de capture de transport et de diffusion de lumière selon l'invention ;

[Fig. 6], une vue schématique en coupe d'un troisième mode de réalisation alternatif d'une cloche du dispositif de capture de transport et de diffusion de lumière selon l'invention ;

[Fig. 7], une vue schématique en plan d'un bassin muni d'une pluralité de dispositifs de capture, de transport et de diffusion de lumière selon l'invention en flottation libre et en circulation aléatoire ;

[Fig. 8], une vue schématique en plan d'un bassin muni d'une pluralité de dispositifs de capture, de transport et de diffusion de lumière selon l'invention en flottation libre et en position fixe organisé dans une structure maillée ; et

[Fig. 9], une vue schématique en coupe d'un cinquième mode de réalisation d'un dispositif de capture, de transport et de diffusion de lumière selon l'invention muni d'un dispositif d'éclairage artificiel rechargeable autonome positionné en extrémité des manches de diffusion de la lumière ;

[Fig. 10], une vue schématique en coupe d'un mode de réalisation alternatif ou complémentaire du dispositif de la figure 9, comprenant un dispositif d'éclairage artificiel rechargeable par induction en alternative ou en combinaison des cellules photoélectriques du dispositif de la figure 9 ;

[Fig. 11], une vue schématique en coupe d'un dispositif de nettoyage spécialement adapté au dispositif de capture, de transport et de diffusion de lumière selon l'invention ;

[Fig. 12], une vue schématique en coupe vue de dessus du dispositif de nettoyage et du dispositif de capture, de transport et de diffusion de lumière de la figure 11 ;

[Fig. 13], une vue schématique en coupe d'un autre mode de réalisation alternatif d'un dispositif de capture, de transport et de diffusion de lumière selon l'invention en position d'utilisation dans un réservoir, et comprenant un réflecteur convexe de lumière à l'extrémité inférieure de la manche ;

[Fig. 14], une vue schématique en coupe d'un mode de réalisation alternatif du dispositif de capture, de transport et de diffusion de lumière de la figure 13, dans lequel le réflecteur est moins large que l'extrémité inférieure de la manche pour laisser passer une partie de la lumière vers le fond du bassin ;

[Fig. 15], une vue schématique en perspective d'un autre mode de réalisation de réflecteur convexe comprenant des trous pour laisser passer une partie de la lumière vers le fond du bassin ;

[Fig. 16], une vue schématique en perspective d'un autre mode de réalisation de réflecteur convexe présentant une surface réfléchissante unie ;

[Fig. 17], une vue schématique en perspective d'un autre mode de réalisation de réflecteur convexe présentant une surface réfléchissante en mosaïque ;

[Fig. 18], une vue schématique en coupe d'un autre mode de réalisation alternatif du dispositif de capture, de transport et de diffusion de lumière de la figure 13, dans lequel le réflecteur est concave ;

[Fig. 19], une vue schématique en coupe du réflecteur concave de la figure 18 présentant une surface réfléchissante unie ;

[Fig. 20], une vue schématique en coupe d'un autre mode de réalisation de réflecteur concave présentant une surface réfléchissante en mosaïque ;

[Fig. 21], une vue schématique en coupe d'une mode de réalisation alternatif de la cloche d'un dispositif de capture, de transport et de diffusion de lumière selon l'invention, constituée de deux parties concentriques destinées à être fixées l'une contre l'autre de manière étanche, en cours d'assemblage ;

[Fig. 22], une vue schématique en coupe de la cloche de la figure 21 après assemblage, et entre les parois de laquelle de la mousse expansée est ajoutée ;

[Fig. 23], une vue schématique en coupe d'une mode de réalisation alternatif de la cloche d'un dispositif de capture, de transport et de diffusion de lumière selon l'invention, comprenant une partie en dôme vitrée destinée à être fixée de manière étanche sur une partie cylindrique en mousse, en cours d'assemblage ; et

[Fig. 24], une vue schématique en coupe de la cloche de la figure 23 après assemblage.

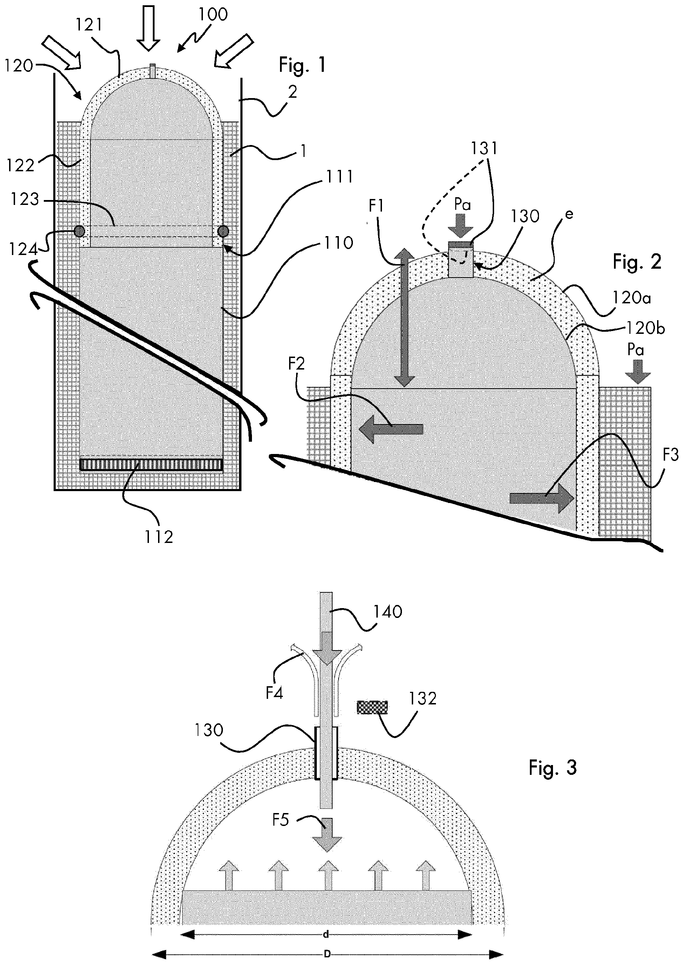

La figure 1 illustre un positif de capture, de transport et de diffusion de lumière 100 selon l'invention flottant librement dans une solution 1 contenue dans un réservoir 2, c'est-à-dire que le dispositif n'est pas fixé à un support immobile par rapport au réservoir.

Le dispositif 100 comprend une manche 110 cylindrique, en matériau souple ou semi-rigide transparent à la lumière transportée et diffusée.

Une extrémité supérieure 111, en position d'utilisation, de la manche 110 est enfilée autour et bloquée contre une cloche 120 étanche, transparente à la lumière transportée et diffusée, et formée par deux parois 120a et 120b séparées par un interstice e rempli en tout ou en partie d'un gaz (air ou autre). Plus particulièrement, la cloche 120 comprend une partie en dôme 121 prolongée par une partie tubulaire 122 sur laquelle est enfilée la manche 110.

Ainsi, l'interstice peut être rempli exclusivement de gaz, ou être rempli partiellement d'une mousse à cellules fermées remplies de gaz. Ce qui importe est que la structure en double paroi permette la flottabilité du dispositif selon l'invention. Dans ce mode de réalisation, le remplissage avec de la mousse devra être partiel et localisé au niveau de la partie tubulaire pour que la portion en dôme reste transparente et laisse passer la lumière vers la manche.

Une extrémité inférieure 112 de la manche est fermée de manière étanche. De préférence, la seconde extrémité 112 de la manche est munie d'une fermeture étanche lestée. Il peut s'agir, par exemple d'une barrette amovible de fermeture étanche lestée.

Alternativement, l'extrémité de la manche peut être soudée de manière à incorporer du leste immergé au fond de la manche.

À titre d'exemple, la cloche est en un matériau transparent rigide, choisi parmi le polypropylène (PP), le poly(méthacrylate de méthyle) (PMMA), le poly(téréphtalate d'éthylène) (PET), ou un plastique transparent traité anti UV.

Également à titre d'exemple, la manche est en un matériau transparent, souple ou semi-rigide, choisi parmi le polyester, le polypropylène (PP), et le polyéthylène téréphtalate, en particulier en un matériau connu sous la marque déposée Mylar®, ou matériau équivalent résistant aux ultraviolets, d'épaisseur de quelques dizaines de micromètres

La manche peut être obtenue en enroulant une portion rectangulaire de feuille et en assemblant les deux bords parallèles par collage ou soudage. L'extrémité inférieure de la manche est fermée tandis que l'extrémité supérieure reste ouverte pour être fixée, sur sa périphérie, à la partie tubulaire 122 de la cloche 120.

La manche cylindrique peut être obtenue par un procédé d'extrusion soufflage sans jointure longitudinale Ainsi, selon l'invention, en position d'utilisation, la manche et la cloche sont remplies d'un liquide propre à transmettre et diffuser la lumière et à assure la pression statique intérieure pour maintenir la manche immergée et étendue verticalement sous la cloche en adoptant une forme cylindrique de sorte que l'ensemble cloche/manche flotte librement dans la solution, au moins la partie en dôme étant maintenue émergée par rapport au niveau du bassin. Cette dernière permet alors non seulement de capter la lumière quelle que soit son angle d'incidence, mais également de maintenir une partie de liquide au-dessus du niveau de la solution, ce qui permet à la manche de garder sa forme tubulaire et cylindrique du fait de la pression statique différentielle exercée sur toute la hauteur de la manche.

La lumière est transportée dans la colonne étanche de liquide (délimitée par la cloche et la manche fermée hermétiquement) qui fait office de « fibre optique » naturelle qui conduit la lumière naturelle depuis l'extérieur vers les profondeurs de la solution algale.

Le dispositif selon l'invention permet donc de capter, transporter et de distribuer la lumière incidente naturelle au sein du volume de culture algale de façon homogène avec un taux maximal de capture du rayonnement, quels que soient son orientation (azimut) et l'angle solaire. À cette fin, le dôme 121 est avantageusement hémisphérique.

Ce dispositif simple et performant permet de valoriser au maximum le potentiel photosynthétique du rayonnement naturel afin accélérer la production des micro algues tout en réduisant significativement le risque d'inhibition biologique et de surchauffe localisée dans la strate supérieure du bassin.

En effet, l'ensemble flottant librement à la surface de la solution, il génère un mélange permanent de la solution algale.

La répartition homogène de la lumière s'accompagne également d'une bonne répartition de la chaleur au sein du volume algal.

La cloche étant constituée d'une double paroi remplie d'un gaz, elle permet non seulement la flottaison, mais également l'isolation thermique du liquide qui ne s'échauffe que très peu.

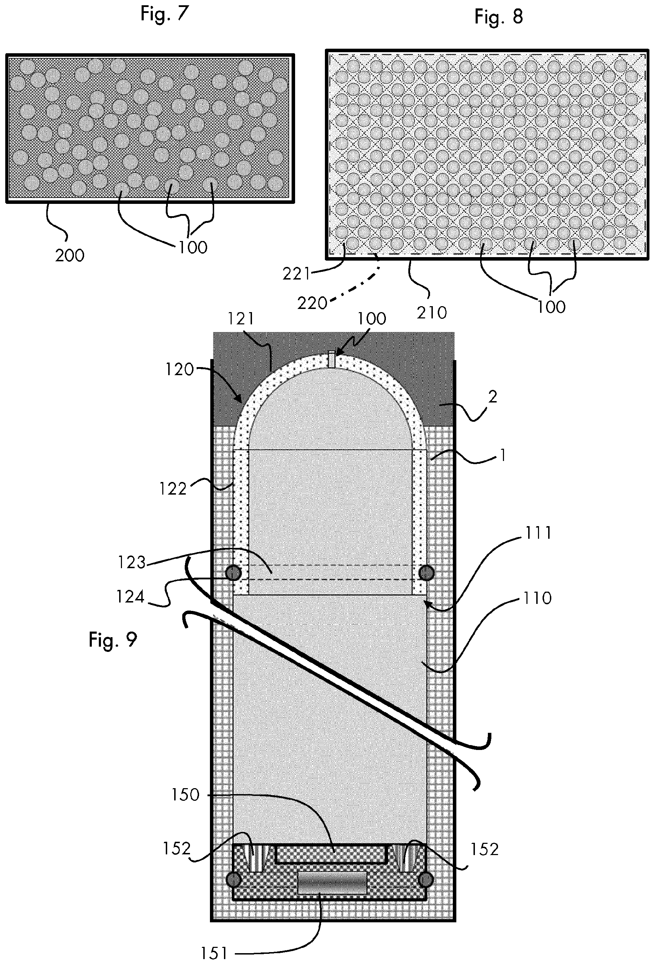

Le dispositif assure ainsi une fonction d'isolation thermique efficace due à qualité d'isolation de la double paroi en lame d'air statique et par la réduction significative de la surface d'évaporation du bassin lorsque plusieurs dispositifs selon l'invention sont utilisés simultanément dans le bassin, comme il sera expliqué en relation avec les figures 7 et 8.

L'intensité du rayonnement incident est répartie dans le rapport de la surface du bassin et de la surface des diffuseurs de lumière, le facteur de répartition peut être très important et défini selon les besoins en photons des différentes souches de micro algues.

La hauteur des manches de transport et de diffusion de la lumière sera adaptée en fonction de l'intensité de rayonnement naturel et des besoins en photons nécessaire à la photo synthèse

De préférence, la partie tubulaire 122 de la cloche 120 comprend une gorge annulaire 123 destinée à recevoir, en utilisation, une bague torique extensible 124 de maintien étanche de la première extrémité 111 de fixation de la manche contre la partie tubulaire.

Selon un mode de réalisation avantageux illustré en figure 2, la partie en dôme 121 comprend un évent tubulaire 130 traversant de manière étanche les deux parois 120a et 120b, reliant ainsi l'extérieur et l'intérieur de la cloche. L'évent 130 comprend une valve 131 de maintien de pression atmosphérique équipée d'une membrane souple étanche qui peut se déformer sous la pression atmosphérique Pa.

Cela permet de maintenir une pression hydrostatique différentielle (flèche F1) constante sur toute la hauteur de la manche.

Au niveau de la partie tubulaire étanche 122 de la cloche 120, la pression de l'air est indépendante de la pression du liquide, elle sera celle de la pression atmosphérique au moment de la fabrication de la cloche (flèches F2 et F3).

Outre la valve souple qui assure l'équilibre de pression avec la pression atmosphérique, l'évent permet un remplissage et une vidange de la cloche et de la manche, comme illustré en figure 3.

À cette fin, on dévisse le bouchon 132 de l'évent (bouchon comprenant l'éventuelle valve 131), et on insère un tube de remplissage/vidange 140, de diamètre inférieur à celui de l'évent de manière à laisser passer l'air en remplissage (flèche F4).

Lorsque le dispositif est rempli de liquide (de préférence de l'eau salée, car cela limite la prolifération bactérienne à l'intérieur de la manche ; flèche F5) jusqu'à l'évent 130, on referme ce dernier en s'assurant qu'aucune bulle d'air n'est enfermée sous la cloche.

Pour éviter tout risque de formation de bulles, le liquide est préalablement dégazé avant de remplir la cloche et la manche.

Si la partie en dôme 121 est avantageusement hémisphérique (c'est-à-dire qu'elle présente un rayon R constant), la partie tubulaire 122 peut avoir différentes formes. Ce qui importe c'est que la manche puisse y être fixée de manière étanche et que le dôme reste émergé pendant l'utilisation pour capter et transmettre la lumière.

La largeur de l'interstice e (et donc le volume de gaz qu'il contient lorsque l'interstice est rempli exclusivement de gaz), la hauteur he de la partie en dôme et la hauteur hi de la partie tubulaire (voir figure 3) sont choisie en fonction des dimensions souhaitées (longueur et diamètre) de la manche et des dimensions du réservoir, de manière à assurer la flottabilité de l'ensemble, tout en assurant l'émergence de la partie en dôme et le maintien de la forme cylindrique de la manche fixée sous la cloche.

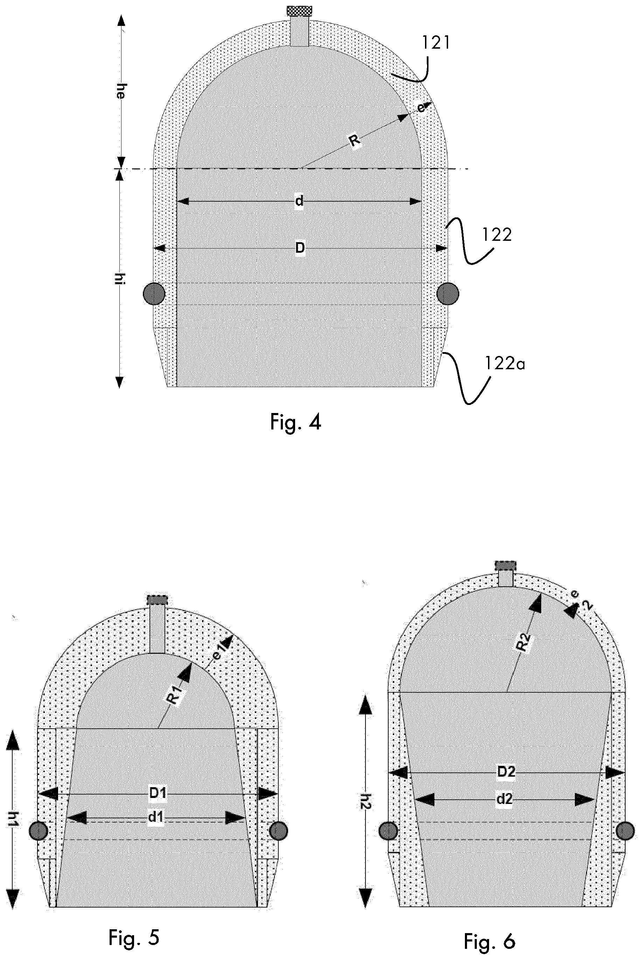

Comme illustré en figure 4, la hauteur émergée he de la cloche est ajustée en définissant le rapport entre le volume V1 d'air enfermé entre les deux parois de la partie tubulaire 122 immergée et le volume V2 de la partie en dôme hémisphérique 121 rempli d'eau

Le volume V1 est calculé de la manière suivante :

[Math. 1]

$$V1 = \frac{\pi}{4} * h * (D - d)$$

Le volume V2 est calculé de la manière suivante :

[Math. 2]

$$V2 = (\frac{4\pi}{3} * R3) \, / \, 2$$

La figure 4 illustre un mode de réalisation avantageux dans lequel l'embase 122a de la partie tubulaire 122 est conique, ce qui permet un enfilage aisé de la manche autour de la cloche. Dans ce mode de réalisation la partie tubulaire est régulière, c'est-à-dire qu'elle présente un diamètre intérieur d et un diamètre extérieur D constants, sauf, bien sûr au niveau de l'embase 122a.

Le tableau 1 suivant indique les différents calculs et données relatives à un exemple de réalisation conforme à la figure 4 :

[Tableaux 1]

| Pour les essais de mesure optique | Unité | Base | V2 | V3 |
|---|---|---|---|---|
| Diamètre externe D | m | 0,15 | 0,15 | 0,15 |
| Épaisseur du vide d'air entre les parois int. et ext. | m | 0,01 | 0,005 | 0,02 |
| Diamètre interne d | m | 0,13 | 0,14 | 0,11 |
| Rayon sphère remplie d'eau | m | 0,065 | 0,07 | 0,055 |
| Hauteur cylindrique immergée | m | 0,15 | 0,35 | 0,05 |
| Hauteur totale de la cloche | m | 0,225 | 0,425 | 0,125 |
| Volume d'eau émergeant | dm3 | 0,575 | 0,718 | 0,348 |
| Volume d'air immergé dans l'anneau cylindrique | dm3 | 0,660 | 0,797 | 0,408 |
| Différence entre le volume immergé et émergeant | dm3 | 0,085 | 0,079 | 0,060 |
| Vérification du volume immergé en air | | OK | OK | OK |
| Échange thermique des cloches | | | | |
| Conductivité thermique matériau | W/m.°C | 0,19 | 0,19 | 0,19 |
| Conductivité thermique air | W/m.°C | 0,023 | 0,023 | 0,023 |
| Épaisseur matériau des 2 parois | mm | 6 | 6 | 6 |
| Épaisseur lame air | mm | 10 | 5 | 20 |
| Échange superficiel ext et interne | 1/he + 1/hi | 0,06 | 0,06 | 0,06 |

(suite)

| Pour les essais de mesure optique | Unité | Base | V2 | V3 |
|---|---|---|---|---|
| Résistivité thermique | °C.m$^2$/W | 0,53 | 0,31 | 0,96 |
| Coefficient d'échange surfacique K | W/°C.m2 | 1,90 | 3,24 | 1,04 |

La figure 5 illustre un mode de réalisation alternatif dans lequel l'interstice de la double paroi présente une largeur e1 plus importante, par exemple si l'on souhaite augmenter la capacité d'isolation thermique de la cloche ou pour gagner du poids ou pour faciliter une fabrication par moulage.

La partie tubulaire présente avantageusement un diamètre interne d1 qui s'élargit vers le bas, c'est-à-dire vers la manche, en position d'utilisation. La fonction d'élargissement du diamètre interne (élargissement faible ou important en fonction de la longueur de la partie tubulaire) dépend de la flottabilité nécessaire pour compenser le volume de la cloche en fonction de la manche choisie.

La figure 6 illustre un mode de réalisation alternatif dans lequel la flottabilité est principalement assurée par la partie tubulaire, la largeur e2 de l'interstice au niveau du dôme étant plus faible que dans la figure 5, l'interstice étant destiné à être rempli exclusivement de gaz dans ce mode de réalisation.

Dans ce cas, le diamètre intérieur d2 de la partie tubulaire se réduit vers le bas, c'est-à-dire vers la manche.

Ce mode de réalisation est particulièrement avantageux lorsque l'on peut limiter le pouvoir isolant de la cloche, car l'augmentation du diamètre des capteurs hémisphériques émergents favorise la capture et la transmission des rayons lumineux vers la solution algale.

Grâce au dispositif selon l'invention, et en particulier à la cloche à double paroi étanche 120, les manches tubulaires cylindriques flottent et peuvent se déplacer librement à la surface de la solution algale.

La figure 7 illustre un réservoir 200 de culture algale vue de dessus, dans lequel flottent librement une multitude de dispositifs 100 selon l'invention. Le nombre de dispositifs 100 est défini en fonction du taux de répartition optimal recherché selon l'intensité du rayonnement naturel et les besoins en photons nécessaires à la photosynthèse.

Grâce au mouvement aléatoire des dispositifs, ils se rencontrent régulièrement, ce qui génère une friction douce périodique entre les manches souples et limite ainsi la formation du biofilm en surface externe.

Pour assurer un déplacement régulier des dispositifs 100 à la surface de la solution algale, le photo-bioréacteur selon l'invention comprend avantageusement des moyens d'agitation (non illustrés) de la solution algale, de manière à générer un courant apte à déplacer les dispositifs de transport et de diffusion de lumière selon l'invention en flottation libre.

De préférence, les moyens d'agitation comprennent un distributeur d'un gaz sous pression, en particulier du dioxyde de carbone, ce qui permet, outre l'agitation, d'alimenter les algues en $CO_2$ et favoriser leur développement.

Alternativement ou en combinaison, les moyens d'agitation peuvent être hydro-mécaniques et comprendre au moins une hélice ou un agitateur rotatif.

Le dispositif selon l'invention permet donc de limiter très fortement l'entretien et le nettoyage. Cependant, le flottage libre aléatoire sur toute la surface du bassin peut conduire à un agglutinement des dispositifs 100 dans une partie du bassin, réduisant la production d'algue dans l'autre partie du bassin qui bénéficie, alors, d'une distribution d'éclairement moindre.

Selon un mode de réalisation avantageux de photo-bioréacteur selon l'invention, illustré en figure 8, on dispose à la surface de la solution, dans le bassin 210, une structure maillée 220 dont chaque maille 221 est de dimension strictement supérieure à un diamètre maximal D, D1, D2 de la cloche des dispositifs 100 de transport et de diffusion de lumière selon l'invention.

Puis on dispose un dispositif de transport et de diffusion de lumière selon l'invention dans chaque maille, de sorte qu'ils peuvent avoir chacun un mouvement aléatoire délimité par la maille dans lequel ils se trouvent.

Plus les dimensions des mailles sont proches du diamètre des cloches, moins les dispositifs selon l'invention parcourent de distance, ce qui assure un frottement très régulier des manches entre elles et évite le développement de biofilm en surface externe. Cependant, les dimensions des mailles ne doivent pas être trop proches de celles des cloches pour permettre la culture d'un volume suffisant d'algues au regard du volume total des dispositifs, et ainsi assurer une bonne productivité du photo-bioréacteur.

Grâce à la structure maillée, il n'y a aucun risque d'agglutinement des dispositifs selon l'invention et la transmission de lumière est homogène sur toute la surface du bassin.

Un mode de réalisation particulièrement avantageux de structure maillée est un filet souple et flottant, fixé au pourtour du bassin 210.

Selon un mode de réalisation optionnel, illustré en figure 9, la manche comprend dans son extrémité inférieure des cellules photovoltaïques 150 dirigées, en utilisation, vers la cloche de capture située à l'extrémité haute, et reliées à un accumulateur 151.

Ainsi, pendant une période d'éclairement fort, une partie de la lumière captée et transmise par la cloche et transportée vers l'extrémité basse de la manche est captée par les cellules photovoltaïques 150 et transformée en électricité accumulée par l'accumulateur 151.

Ce dernier est relié à des diodes électroluminescentes 152 dirigées, en utilisation, vers le haut la cloche.

Ainsi, la nuit ou pendant des périodes de faible intensité solaire, l'accumulateur est programmé pour alimenter les diodes luminescentes qui vont éclairer l'intérieur de la manche. En raison de l'agencement du dispositif, la lumière ne va pas sortir de la cloche, mais être réfléchie vers tout le dispositif qui va transmettre et distribuer la lumière vers la solution algale du bassin.

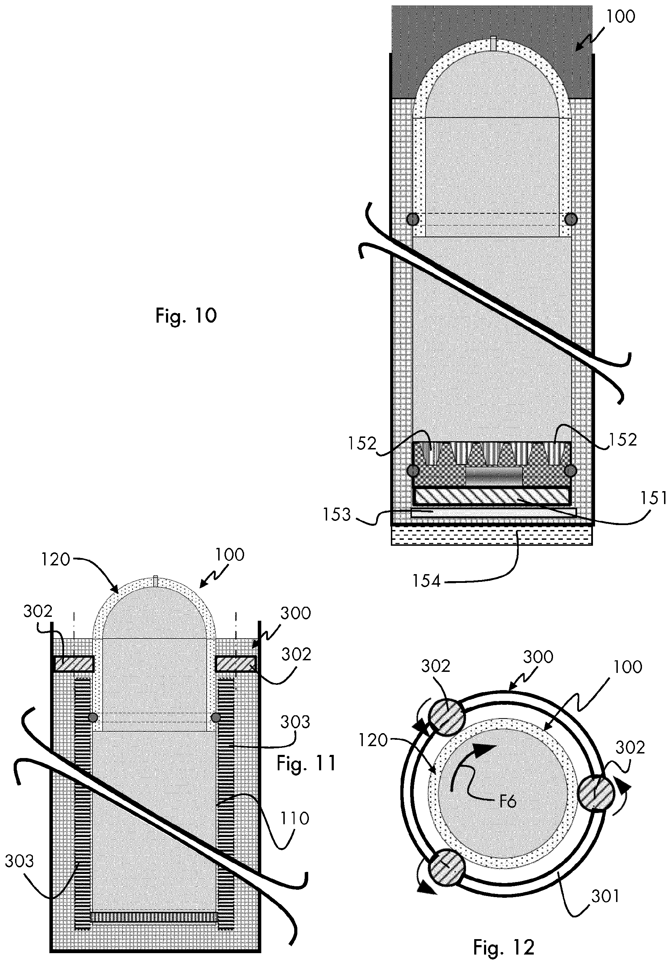

Alternativement ou en combinaison, un mode de réalisation avantageux permettant un rechargement de l'accumulateur par induction sans fil est illustré en figure 10. Dans ce mode de réalisation, l'accumulateur 151 est relié à un résonateur récepteur de puissance 153 capable de transformer en électricité l'énergie magnétique transmise par des résonateurs émetteurs de puissance 154 équipant le fond du bassin.

Leur nombre et leur puissance émettrice varient en fonction des dispositifs en circulation dans le bassin
Le chargement des accumulateurs a lieu de préférence pendant les périodes tarifaires favorables, la nuit et le week-end.

Ce mode de réalisation permet, en outre, d'alimenter les diodes électroluminescentes en période nocturne ou de faible ensoleillement, lorsque les accumulateurs chargés en journée, pendant les périodes de fort ensoleillement, se sont épuisés.

Ce dispositif de chargement par induction peut remplacer les cellules photoélectriques 150 du mode de réalisation de la figure 9 ou bien les compléter.

Malgré la très grande efficacité du dispositif selon l'invention pour limiter le développement de biofilm à la surface extérieure des manches, il peut être souhaitable de pouvoir nettoyer les manches périodiquement et facilement.

Or un tel nettoyage est une étape très complexe dans les photo-bioréacteurs de l'art antérieur, car il démonter le support et toutes les manches en même temps, ce qui est complexe, risqué et interrompt la production.

Le dispositif selon l'invention, grâce à sa structure spécifique et à sa liberté de mouvement permet un nettoyage aisé sans interrompre le fonctionnement des autres dispositifs présents dans le bassin, et ce de manière très rapide et automatisable, ce qui est un avantage important en termes d'exploitation.

L'invention propose ainsi un dispositif de nettoyage automatique escamotable d'un dispositif de capture, de transport et de diffusion de lumière selon l'invention, qui peut être installé périodiquement dans le bassin du photo-bioréacteur selon l'invention.

Ce dispositif de nettoyage 300 est illustré, en position de nettoyage, en figure 11.

Il comprend un support circulaire 301 munie de galets motorisés rotatifs 302 pour l'entraînement en rotation de la cloche 120 du dispositif 100 de capture, de transport et de diffusion de lumière selon l'invention.

Le support comprend, en outre, au moins une brosse 303 agencée sous le support 301 et en contact avec la manche 110 du dispositif 100 de capture, de transport et de diffusion de lumière selon l'invention, en position de nettoyage.

Avantageusement, le support comprend également des flotteurs pour maintenir le dispositif de nettoyage autour des dispositifs de capture, de transport et de diffusion de lumière selon l'invention.

Ainsi, en utilisation, le dispositif de nettoyage est positionné et introduit pardessus le dispositif de capture, de transport et de diffusion de lumière selon l'invention, de telle sorte que le support soit agencé autour de la cloche, que le ou les galets soient en contact avec la cloche et que la ou les brosses soient en contact avec la manche.

Puis, comme illustrés en figure 12, le ou les galets sont actionnés et entraînent en rotation (flèche F6) le dispositif de capture, de transport et de diffusion de lumière, par friction contre la cloche du dispositif. Ce faisant, la brosse racle la face extérieure de la manche et la débarrasse du biofilm et de toute autre saleté très rapidement et automatiquement.

Dans un mode de réalisation non illustré, le support peut être constitué en deux demi-cercles articulés l'un par rapport à l'autre, pour permettre un agencement de manière latérale par rapport au dispositif de capture, de transport et de diffusion de lumière selon l'invention.

Ainsi le nettoyage peut être réalisé in situ dans le bassin, en peu de temps par dispositif, sans démontage des dispositifs de capture, de transport et de diffusion de lumière selon l'invention, ni arrêt d'exploitation des autres dispositifs, avec un ou plusieurs dispositifs de nettoyage en fonctionnement simultané mis en œuvre par un seul opérateur

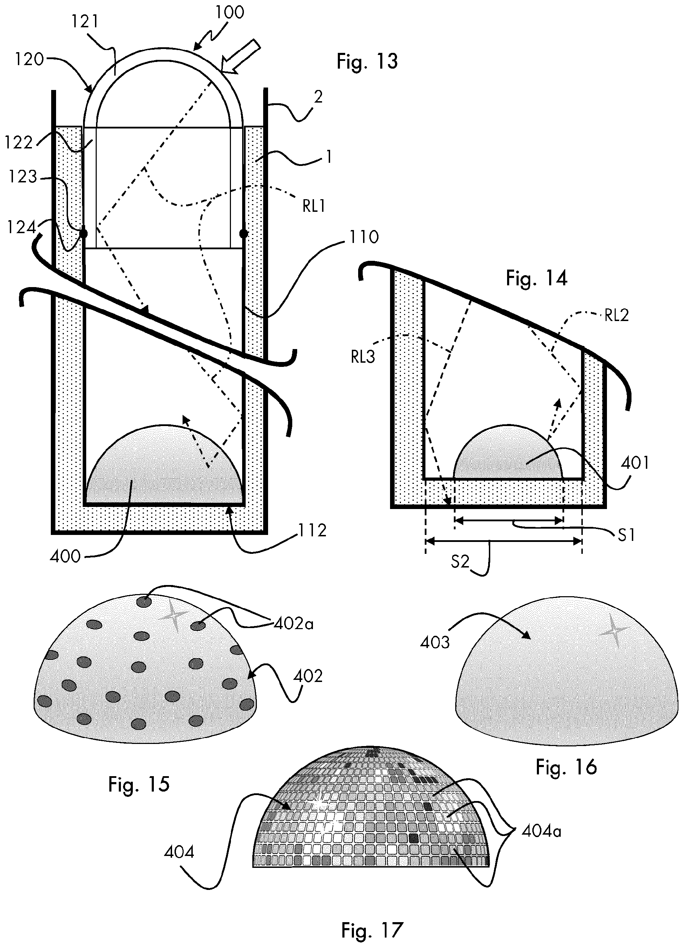

La figure 13 illustre un mode de réalisation avantageux qui augmente l'efficacité de la transmission de lumière à la solution d'algues.

Comme expliqué précédemment, la différence d'indice optique entre le liquide contenu dans la manche et la solution algale permet de conduire la lumière provenant du dôme 121 vers l'extrémité inférieure de la manche à la manière d'une fibre optique.

Cependant, dans certaines conditions (par exemple lorsque la solution algale devient très concentrée en algues), il peut arriver qu'une trop grande quantité de lumière soit acheminée vers cette extrémité inférieure par rapport à la quantité de lumière qui arrive à s'échapper par les parois latérales de la manche. Cela conduit à un spot de lumière assez dense au fond du bassin qui peut être néfaste pour les algues.

Selon le mode de réalisation illustré en figure 13, l'extrémité inférieure 112 de la manche 110 comprend un réflecteur 400 conformé pour réfléchir la lumière vers la manche, et notamment vers les parois latérales et l'extrémité supérieure de la manche.

Grâce au réflecteur 400, un rayon lumineux RL1 provenant du dôme 121 et conduit le long de la manche 110 est réfléchi vers la manche plutôt que de s'échapper vers le bassin par l'extrémité inférieure 112 de la manche 110.

Dans un mode de réalisation avantageux, illustré en figure 14, le réflecteur 401 présente une surface de contact S1 avec l'extrémité inférieure 112 de la manche, plus petite que la surface S2 de ladite extrémité inférieure 112 de la manche.

De cette manière, une partie de la lumière, matérialisée par le rayon lumineux RL2 peut passer au travers de l'extrémité inférieure de la manche, alors qu'une autre partie de la lumière, matérialisée par le rayon lumineux RL3 est réfléchi par le réflecteur 401.

Alternativement ou en combinaison, le réflecteur 402 (voir figure 15) est ajouré et présente des trous 402a à sa surface de manière à laisser passer une partie de la lumière vers l'extrémité inférieure de la manche.

Le nombre de trous, leur place et leur surface sont modulés en fonction de la quantité de lumière que l'on souhaite laisser passer au travers de l'extrémité inférieure 112 de la manche 110.

Les figures 16 et 17 illustrent des réflecteurs 403-404 présentant deux types de surfaces réfléchissantes différentes. Le premier réflecteur 403, illustré en figure 16, présente une surface réfléchissante unie, de type miroir. Ce mode de réalisation permet une réflexion uniforme de la lumière dans toutes les directions, sur 180°.

Le deuxième type de réflecteur 404, illustrée en figure 17, présente une surface réfléchissante en mosaïque, c'est-à-dire comprenant une multitude de petites facettes planes 404a. Ce mode de réalisation permet une réflexion plus intense dans certaines directions (au niveau des facettes), de manière à concentrer la lumière et permettre une meilleure transmission au travers des parois latérales de la manche 110.

Le réflecteur selon l'invention est avantageusement hémisphérique ou sensiblement hémisphérique pour réfléchir les rayons lumineux vers les parois latérales de la manche et ainsi favoriser leur transmission vers la solution algale.

De préférence, le réflecteur est convexe (figures 13 à 17), c'est-à-dire que sa surface réfléchissante extérieure est convexe. Ceci permet à la fois une bonne réflexion des rayons lumineux qui l'atteignent, mais également de maintenir la longueur de la manche.

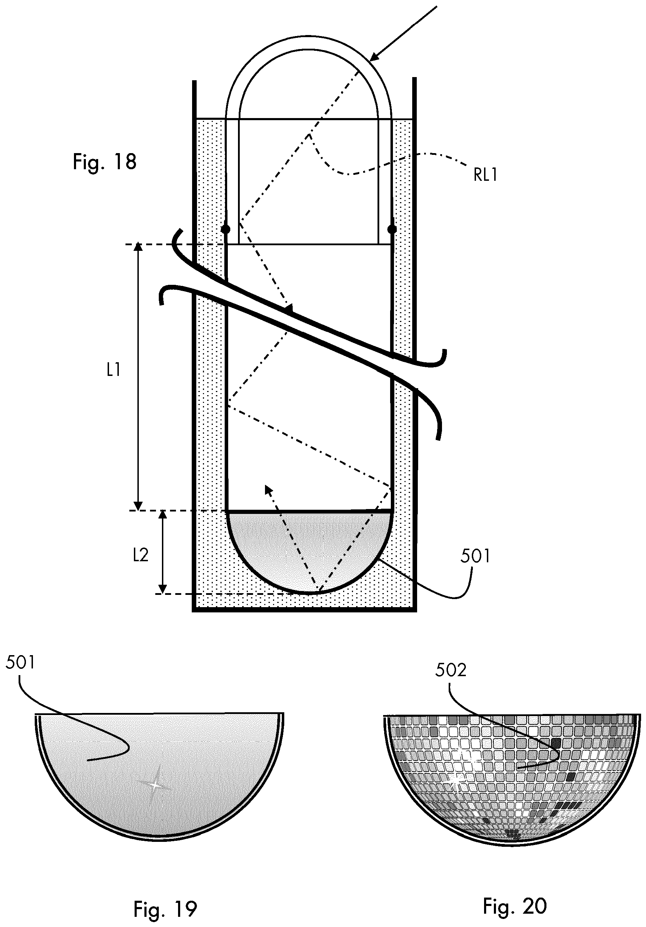

Alternativement, le réflecteur peut être concave (figures 18 à 20), c'est-à-dire que c'est sa face intérieure concave qui est réfléchissante. Ainsi, le réflecteur concave 501 (figures 18-19) présente une face intérieure concave unie, de type miroir. Alternativement, le réflecteur concave 502 (figure 20) présente une face intérieure concave en mosaïque, c'est-à-dire comprenant une multitude de petites facettes planes.

Dans ce cas, la longueur L1 du dispositif selon l'invention est augmentée du rayon L2 du réflecteur, de sorte que toute la surface de la manche peut transmettre des rayons lumineux à la solution algale. Lorsque le réflecteur est convexe, la portion de la manche située juste autour risque de transmettre un peu moins de rayons lumineux, ce qui peut être souhaitable dans certaines situations ou configurations.

Ces deux modes de réalisation peuvent permettre une conception aisée si l'extrémité inférieure 112 de la manche est simplement enfilée autour du réflecteur et maintenue dans cette position de manière étanche, par exemple par un système de gorge et cordon annulaire (non illustré) similaire à ce qui a été décrit pour la fixation de l'extrémité supérieure de la manche sur la cloche. Un réflecteur ajouré concave ou convexe peut également être utilisé dans cette configuration, pourvu que les trous dans la surface réfléchissante soient fermés de manière étanche par une couche d'un matériau transparent aux rayons lumineux.

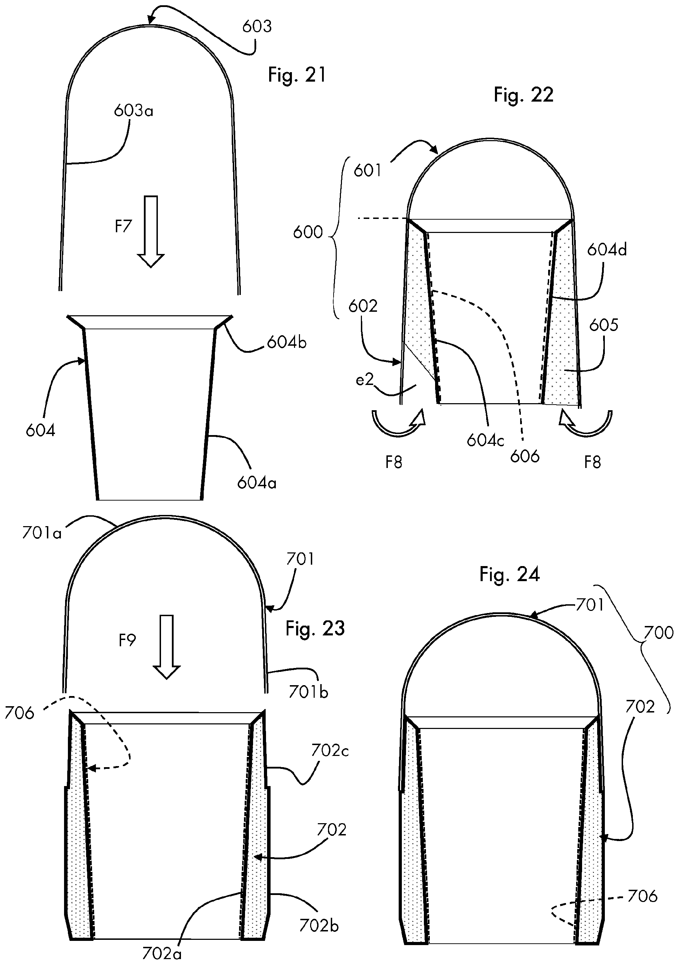

Les figures 21 à 24 illustrent des modes de réalisation avantageux permettant une fabrication facilitée et économique d'un dispositif de capture, de transport et de diffusion de lumière selon l'invention.

Le mode de réalisation illustré aux figures 21 et 22 comprend une cloche 600 formée en partie seulement par deux parois séparées par un interstice étanche : la partie en dôme 601 de la cloche est à simple paroi et la partie tubulaire 602 est à double paroi. Comme le montre la figure 22, c'est la partie tubulaire 602 à double paroi qui, comme dans les modes de réalisation précédents, assure la fonction de flottabilité du dispositif selon l'invention.

Dans ce mode de réalisation, la cloche 600 comprend une première pièce 603 formée d'une seule paroi et comprenant la partie en dôme 601 prolongée par une portion cylindrique. Ce qui importe c'est que la partie en dôme 601 soit transparente aux rayons lumineux, le reste n'étant pas nécessairement transparent. Cependant, pour des raisons de simplicité, la pièce 603 est avantageusement en un seul matériau transparent. De préférence, cette pièce 603 est moulée en un polymère transparent tel que le polypropylène (PP), le poly(méthacrylate de méthyle) (PMMA), le poly(téréphtalate d'éthylène) (PET), ou autre matériau plastique transparent traité anti UV.

La cloche 600 comprend également une seconde pièce 604 comprenant une portion cylindrique 604a munie d'un col 604b destiné, en position d'utilisation, à être en contact périphérique étanche avec une surface intérieure de la première pièce 603.

De cette manière, la combinaison des pièces 603 et 604 (illustrée en figure 22) permet de délimiter un interstice e2 devant être étanche, en utilisation, entre la portion cylindrique de la première pièce et la portion cylindrique de la seconde pièce.

Pour cela, comme illustré en figure 21, la pièce 603 est insérée au-dessus de la pièce 604 de manière concentrique, dans le sens de la flèche F7, jusqu'à ce que le col 604b soit en contact périphérique contre la surface intérieure 603a de la première pièce 603, puis l'interstice e2 est rempli, dans le sens des flèches F8, d'une mousse expansive 605 à cellules fermées contenant du gaz. Ainsi, lors de l'expansion et du séchage, la mousse 605 colle les deux pièces 603 et 604 et rend étanche le contact périphérique entre le col 604b et la surface intérieure 603a de la première pièce 603, tout en assurant la flottabilité du dispositif selon l'invention,

En d'autres termes, la mousse assure deux fonctions, à savoir l'étanchéité de l'interstice e2 et la flottabilité, et permet une fabrication aisée, car cette étape de collage est facile à réaliser et ne nécessite pas une précision particulière comme avec un collage ou une soudure thermique. Si de la mousse déborde au niveau du col 604b, il suffit de la retirer lors de la finition de la pièce.

Une mousse particulièrement intéressante à utiliser est le polyuréthane, car il comprend des cellules fermées et il est étanche. En outre, il est capable de coller fortement les deux pièces 603 et 604.

D'autres composants peuvent être utilisés, du moment que l'on obtient une flottabilité suffisante, que le liquide contenu dans la manche et la cloche ne peut pas fuir et que la lumière peut traverser la partie en dôme.

Lorsque l'interstice n'est rempli qu'en partie par du gaz, il n'est généralement plus ou pas transparent aux rayons lumineux et, surtout, ces derniers risquent de mal se réfléchir contre cette partie de la cloche, de sorte que la conduction de la lumière risque d'en être affectée.

Pour résoudre ce problème, l'invention propose de disposer une couche réfléchissante 606 sur la surface intérieure 604c de la pièce 604 pour réfléchir, en utilisation, la lumière provenant de la partie en dôme vers la manche.

Si cette dernière est constituée par un matériau transparent, il est bien sûr possible de prévoir cette couche réfléchissante contre la surface extérieure 604d de la pièce 604 à la manière d'un miroir, de sorte que la deuxième pièce présente une surface réfléchissante pour réfléchir, en utilisation, la lumière provenant de la partie en dôme vers la manche. Dans ce cas, la couche réfléchissante est appliquée contre la paroi extérieure 604d avant de remplir l'interstice de mousse. De préférence, cela se fait au cours de la fabrication de la pièce 604 elle-même.

Le mode de réalisation illustré aux figures 21-22 présente un avantage important en termes de fabrication, car les pièces sont faciles à fabriquer et à démouler. Pour cela, on prévoit avantageusement que leur forme générale soit évasée pour faciliter le démoulage. En outre, cela permet de ménager un interstice de volume suffisant pour assurer la flottabilité du dispositif final en utilisation, comme expliqué précédemment.

Les figures 23 et 24 illustrent un autre mode de réalisation avantageux, tant pour le fonctionnement que pour la fabrication.

La cloche 700 comprend également deux pièces destinées à être fixées l'une à l'autre lors de la fabrication.

La première pièce 701 ne comprend qu'une paroi et comprend une partie en dôme 701a prolongée par une portion cylindrique 701b légèrement évasée et destinée à coopérer avec la seconde pièce 702. Cela permet un démoulage aisé de la pièce 701, mais assure également un maintien de la pièce 701 contre la pièce 702, comme ce sera expliqué par la suite.

La seconde pièce 702 comprend une portion cylindrique tubulaire constituée, quant à elle, d'un anneau en un matériau à cellules fermées remplies de gaz (par exemple une mousse expansée solidifiée à cellules fermées), présentant une surface interne 702a et une surface externe 702b. Ces deux surfaces constituent une double paroi virtuelle délimitant donc un interstice virtuel dans lequel le matériau enferme du gaz dans les cellules fermées assurant ainsi la fonction de flottabilité.

Ainsi, ce qui importe c'est la double fonction de captation de la lumière par le dôme transparent, et de flottabilité de sorte qu'en position d'utilisation, le dispositif flotte librement dans la solution, que la partie en dôme soit maintenue émergée et que la manche remplie de liquide soit immergée et étendue verticalement sous la cloche.

La portion cylindrique tubulaire est munie d'un épaulement 702c destiné à être en contact périphérique étanche avec la portion cylindrique 701b de la première pièce 701, en position d'utilisation.

Cet anneau 702 est avantageusement moulé de manière à ménager l'épaulement périphérique 702c contre lequel les bords libres 701b du dôme 701 viennent en appui lorsque la pièce 701 est insérée au-dessus de la pièce 702 dans le sens de la flèche F9.

De préférence, l'épaulement est légèrement tronconique (en coupe), ce qui assure un contact étroit avec la portion cylindrique 701b de la première pièce 701 qui est légèrement évasée.

Grâce à cette structure, le dôme est maintenu fermement et de manière étanche contre l'anneau lorsque la cloche et la manche sont remplies de liquide, car ce dernier tend à repousser l'anneau contre le dôme.

Par sécurité, il peut être préférable, néanmoins, de prévoir un moyen de fixation étanche entre les pièces 701 et 702, telle qu'une colle.

Comme dans le mode de réalisation précédent, il est avantageusement prévu que la portion cylindrique de la deuxième pièce 702 présente une surface réfléchissante 706 pour réfléchir, en utilisation, la lumière provenant de la partie en dôme vers la manche.

Le dispositif selon l'invention est insensible aux mouvements verticaux de houle et du vent, car il n'est pas fixé à un support, mais il flotte librement ; il suit donc les mouvements verticaux sans contrainte mécanique, ce qui permet de l'utiliser en étang et en pleine mer sans risque de l'abîmer.

Le dispositif selon l'invention peut équiper des bassins existants de type « raceway » afin d'augmenter significativement leur productivité. Des bassins ouverts de grosse capacité et de plus grande profondeur permettront d'optimiser la production spécifique (objectif de productivité supérieur à 200 t/ha.an de matière séche) et de réduire le coût global foncier, d'équipement et d'installation.

Un usage en bassin extensif naturel, en étang ou en pleine mer est envisageable avec une cloison périphérique flottante, comme pour l'élevage des poissons en mer.

Il est également envisageable d'augmenter le développement des micro algues à proximité des parcs d'ostréiculture et de conchiculture.

**Revendications**

1. Dispositif (100) de capture, de transport et de diffusion de lumière dans une solution (1) contenue dans un réservoir (2, 200, 210), le dispositif comprenant une manche cylindrique (110), en matériau transparent à la lumière transportée et diffusée, la manche (110) étant destinée à être remplie d'un liquide propre à transmettre et diffuser la lumière et à équilibrer la pression extérieure pour maintenir la manche sous forme cylindrique, **caractérisé en ce qu'**il comprend, en outre, une cloche (120) étanche, transparente à la lumière captée, transportée et diffusée, et formée au moins en partie par deux parois (120a-120b) séparées par un interstice étanche (e) rempli en tout ou en partie par du gaz, la cloche comprenant une partie en dôme (121) prolongée par une partie tubulaire (122), une première extrémité (111) de la manche (110) étant destinée à être bloquée contre la cloche (120) de sorte qu'en position d'utilisation, la cloche flotte librement dans la solution et maintient au moins la partie en dôme (121) émergée et la manche (110) remplie de liquide immergée et étendue verticalement sous la cloche en adoptant une forme cylindrique.

2. Dispositif selon la revendication 1, dans lequel la partie en dôme de la cloche est hémisphérique.

3. Dispositif selon la revendication 1 ou 2, dans lequel la partie en dôme de la cloche est à double paroi.

4. Dispositif selon la revendication 1 ou 2, dans lequel la partie en dôme (603, 701a) de la cloche est à simple paroi et la partie tubulaire (602, 702) est à double paroi.

5. Dispositif selon l'une quelconque des revendications 1 à 4, dans lequel l'interstice étanche (e) séparant les doubles parois est rempli exclusivement de gaz.

6. Dispositif selon l'une quelconque des revendications 1 à 4, dans lequel l'interstice étanche (e2) séparant les doubles parois est rempli d'une mousse expansée (605, 702) à cellules fermées remplies de gaz, telle qu'une mousse en polyuréthane.

7. Dispositif selon l'une quelconque des revendications 1 à 6, dans lequel la partie en dôme comprend un évent tubulaire (130) traversant la partie en dôme de manière étanche et comprenant une valve (131) de maintien de pression atmosphérique équipée d'une membrane souple étanche qui peut se déformer sous la pression atmosphérique.

8. Dispositif selon l'une quelconque des revendications 1 à 7, dans lequel la partie tubulaire de la cloche comprend une gorge annulaire (123) destinée à recevoir, en utilisation, une bague torique extensible (124) de maintien étanche d'une première extrémité de fixation de la manche contre la partie tubulaire.

9. Dispositif selon l'une quelconque des revendications 1 à 8 dans lequel la partie tubulaire de la cloche présente une surface conique (604a, 702a) se rétrécissant vers la partie en dôme.

10. Dispositif selon la revendication 4, dans lequel la cloche (600) comprend une première pièce (603) comprenant la partie en dôme (601) prolongée par une portion cylindrique, et une seconde pièce (604) comprenant une portion cylindrique (604a) munie d'un col (604b) destiné à être en contact périphérique étanche avec une surface intérieure (603a) de la première pièce, en position d'utilisation, afin de délimiter un interstice (e2) étanche, en utilisation, entre la portion cylindrique de la première pièce et la portion cylindrique de la seconde pièce.

11. Dispositif selon la revendication 4, dans lequel la cloche (700) comprend une première pièce (701) comprenant la partie en dôme (701a) prolongée par une portion cylindrique (701b), et une seconde pièce (702) comprenant une portion cylindrique (702) munie d'un épaulement (702c) destiné à être en contact périphérique étanche avec la portion cylindrique (701b) de la première pièce, en position d'utilisation, la seconde pièce étant constituée par un anneau en un matériau à cellules fermées remplies de gaz.

12. Dispositif selon la revendication 10 ou 11, dans lequel la portion cylindrique de la deuxième pièce présente une surface réfléchissante (606, 706) pour réfléchir, en utilisation, la lumière provenant de la partie en dôme vers la manche.

13. Dispositif selon l'une quelconque des revendications 1 à 12, dans lequel la manche comprend une seconde extrémité munie d'une fermeture étanche lestée (112).

14. Dispositif selon l'une quelconque des revendications 1 à 13, dans lequel une extrémité inférieure de la manche comprend des cellules photovoltaïques (150) dirigées, en utilisation, vers la cloche, et reliées à un accumulateur (151) relié à des diodes électroluminescentes (152) dirigées, en utilisation, vers la cloche, l'accumulateur étant programmé pour accumuler de l'électricité le jour et pour alimenter les diodes luminescentes la nuit ou pendant des périodes de faible intensité solaire.

15. Dispositif selon la revendication 14, dans lequel l'accumulateur (151) est relié à un résonateur récepteur de puissance (152) capable de transformer en électricité l'énergie magnétique transmise, en position d'utilisation, par des résonateurs émetteurs de puissance (152) équipant le fond du réservoir (2, 200, 210).

16. Dispositif selon l'une quelconque des revendications 1 à 15, dans lequel l'extrémité inférieure (112) de la manche comprend un réflecteur (400, 401, 402, 403, 404, 501, 502) conformé pour réfléchir la lumière vers la manche.

17. Dispositif selon la revendication 16, dans lequel le réflecteur (400, 401, 402, 403, 404) est convexe.

18. Dispositif selon la revendication 16, dans lequel le réflecteur (501, 502) est concave.

19. Dispositif selon l'une quelconque des revendications 16 à 18, dans lequel le réflecteur présente une surface (S1) de contact avec l'extrémité inférieure de la manche inférieure à une surface (S2) de ladite extrémité inférieure de la manche de manière à laisser passer une partie de la lumière au travers de l'extrémité inférieure de la manche.

20. Dispositif selon l'une quelconque des revendications 16 à 18, dans lequel le réflecteur (402) est ajouré de manière à laisser passer une partie de la lumière vers l'extrémité inférieure de la manche.

21. Dispositif selon l'une quelconque des revendications 1 à 20, dans lequel la cloche est en un matériau choisi parmi le polypropylène (PP), le poly(méthacrylate de méthyle) (PMMA), le poly(téréphtalate d'éthylène) (PET), et un plastique transparent traité anti UV.

22. Dispositif selon l'une quelconque des revendications 1 à 21, dans lequel la manche est en un matériau choisi parmi le polyester, le polypropylène (PP) et tout autre matériau transparent en film, d'épaisseur de quelques dizaines de micromètres.

23. Dispositif selon l'une quelconque des revendications 1 à 22, dans lequel la manche, en utilisation, est remplie d'eau salée et préalablement dégazée.

24. Photo-bioréacteur pour la production d'une solution algale concentrée comportant un réservoir (2, 200, 210) contenant la solution algale, **caractérisé en ce qu'**il comprend au moins un dispositif (100) de capture, de transport et de diffusion de lumière selon l'une quelconque des revendications 1 à 23 en flottage libre à la surface de la solution algale.

25. Photo-bioréacteur selon la revendication 24, comprenant, en outre, des moyens d'agitation de la solution algale de capables de générer un courant apte à déplacer des dispositifs de capture, de transport et de diffusion de lumière selon l'une quelconque des revendications 1 à 24 en flottage libre à la surface de la solution.

26. Photo-bioréacteur selon la revendication 25, dans lequel les moyens d'agitation comprennent un distributeur d'un gaz sous pression, en particulier du dioxyde de carbone.

27. Photo-bioréacteur selon l'une quelconque des revendications 13 à 26, comprenant, en outre, une structure maillée (220) dont chaque maille (221) est de dimension strictement supérieure à un diamètre maximal (D, D1, D2) de la cloche dudit au moins un dispositif de transport et de diffusion de lumière, de manière à permettre un mouvement aléatoire délimité d'un dispositif de transport et de diffusion de lumière compris dans une maille.

28. Photo-bioréacteur selon la revendication 27, dans lequel la structure maillée est constituée par un filet souple et flottant.

29. Photo-bioréacteur selon l'une quelconque des revendications 22 à 28, comprenant, en outre, des résonateurs émetteurs de puissance (152) équipant le fond du réservoir (2, 200, 210).

30. Dispositif de nettoyage (300) d'un dispositif de capture, de transport et de diffusion de lumière selon l'une quelconque des revendications 1 à 23, **caractérisé en ce qu'**il comprend un support circulaire (301) munie d'au moins un galet motorisé rotatif (302) pour l'entraînement en rotation de la cloche (120) du dispositif (100) de capture, de transport et de diffusion de lumière en position de nettoyage, le support comprenant, en outre, au moins une brosse (303) agencée sous le support et destinée à être en contact avec la manche (110) du dispositif (100) de capture, de transport et de diffusion de lumière en position de nettoyage.

31. Dispositif de nettoyage (300) selon la revendication 30, dans lequel le support (301) comprend, en outre, des flotteurs.

32. Photo-bioréacteur selon l'une quelconque des revendications 24 à 29, comprenant, en outre, au moins un dispositif de nettoyage selon l'une quelconque des revendications 30 ou 31.

**Patentansprüche**

1. Vorrichtung (100) zum Auffangen, Transportieren und Verbreiten von Licht in einer Lösung (1), die in einem Vorratsbehälter (2, 200, 210) enthalten ist, wobei die Vorrichtung einen zylindrischen Mantel (110) aus für das transportierte und verbreitete Licht transparentem Material umfasst, wobei der Mantel (110) bestimmt ist, mit einer Flüssigkeit gefüllt zu sein, die imstande ist, das Licht zu übertragen und zu verbreiten und den Außendruck auszugleichen, um den Mantel in zylindrischer Form zu halten, **dadurch gekennzeichnet, dass** sie ferner eine dichte, für das aufgefangene, transportierte und verbreitete Licht transparente und mindestens zum Teil von zwei separaten Wänden (120a-120b), die durch einen dichten Spalt (a) getrennt sind, der ganz oder teilweise mit Gas gefüllt ist, Glocke (120) umfasst, wobei die Glocke einen Kuppelteil (121) umfasst, der von einem rohrförmigen Teil (122) verlängert wird, wobei ein erstes Ende (111) des Mantels (110) bestimmt ist, an der Glocke (120) arretiert zu sein, so dass die Glocke in Benutzungsposition frei in der Lösung schwimmt und mindestens den Kuppelteil (121) aufgetaucht und den mit Flüssigkeit gefüllten Mantel (110) eingetaucht und vertikal erstreckt unter der Glocke bei Annahme einer zylindrischen Form hält.

2. Vorrichtung nach Anspruch 1, wobei der Kuppelteil der Glocke halbkugelförmig ist.

3. Vorrichtung nach Anspruch 1 oder 2, wobei der Kuppelteil der Glocke doppelwandig ist.

4. Vorrichtung nach Anspruch 1 oder 2, wobei der Kuppelteil (603, 701a) der Glocke einwandig ist und der rohrförmige Teil (602, 702) doppelwandig ist.

5. Vorrichtung nach einem der Ansprüche 1 bis 4, wobei der dichte Spalt (e), der die doppelten Wände trennt, ausschließlich mit Gas gefüllt ist.

6. Vorrichtung nach einem der Ansprüche 1 bis 4, wobei der dichte Spalt (e2), der die doppelten Wände trennt, mit einem expandierten Schaum (605, 702) mit geschlossenen, mit Gas gefüllten Zellen, wie ein Polyurethanschaum, gefüllt ist.

7. Vorrichtung nach einem der Ansprüche 1 bis 6, wobei der Kuppelteil eine rohrförmige Entlüftung (130) umfasst, die den Kuppelteil dicht durchquert und ein Ventil (131) zwecks Erhalts des atmosphärischen Drucks umfasst, das mit einer elastischen dichten Membran ausgestattet ist, die sich unter dem atmosphärischen Druck verformen kann.

8.  Vorrichtung nach einem der Ansprüche 1 bis 7, wobei der rohrförmige Teil der Glocke eine ringförmige Nut (123) umfasst, die in Benutzung zur Aufnahme eines dehnbaren torischen Rings (124) zum Dichthalten eines ersten Befestigungsendes des Mantels am rohrförmigen Teil bestimmt ist.

9.  Vorrichtung nach einem der Ansprüche 1 bis 8, wobei der rohrförmige Teil der Glocke eine konische Oberfläche (604a, 702a) aufweist, die sich zum Kuppelteil verengt.

10. Vorrichtung nach Anspruch 4, wobei die Glocke (600) ein erstes Teil (603), das den von einem zylindrischen Abschnitt verlängerten Kuppelteil (601) umfasst, und ein zweites Teil (604) umfasst, das einen zylindrischen Abschnitt (604a) umfasst, der mit einem Kragen (604b) ausgestattet ist, der bestimmt ist, in Benutzungsposition mit einer inneren Oberfläche (603a) des ersten Teils in dichtem Umfangskontakt zu sein, um in Benutzung einen dichten Spalt (e2) zwischen dem zylindrischen Abschnitt des ersten Teils und dem zylindrischen Abschnitt des zweiten Teils zu begrenzen.

11. Vorrichtung nach Anspruch 4, wobei die Glocke (700) ein erstes Teil (701), das den von einem zylindrischen Abschnitt (701b) verlängerten Kuppelteil (701a) umfasst, und ein zweites Teil (702) umfasst, das einen zylindrischen Abschnitt (702) umfasst, der mit einem Absatz (702c) ausgestattet ist, der bestimmt ist, in Benutzungsposition mit dem zylindrischen Abschnitt (701b) des ersten Teils in dichtem Umfangskontakt zu sein, wobei das zweite Teil aus einem Ring aus einem Material mit geschlossenen, mit Gas gefüllten Zellen besteht.

12. Vorrichtung nach Anspruch 10 oder 11, wobei der zylindrische Abschnitt des zweiten Teils eine reflektierende Oberfläche (606, 706) aufweist, um in Benutzung das vom Kuppelteil kommende Licht zum Mantel zu reflektieren.

13. Vorrichtung nach einem der Ansprüche 1 bis 12, wobei der Mantel ein zweites Ende umfasst, das mit einem ballastierten dichten Verschluss (112) ausgestattet ist.

14. Vorrichtung nach einem der Ansprüche 1 bis 13, wobei ein unteres Ende des Mantels Photovoltaikzellen (150) umfasst, die in Benutzung zu der Glocke gerichtet sind und mit einem Akkumulator (151) verbunden sind, der mit elektrolumineszierenden Dioden (152) verbunden ist, die in Benutzung zu der Glocke gerichtet sind, wobei der Akkumulator programmiert ist, um am Tag Elektrizität zu akkumulieren und um die lumineszierenden Dioden in der Nacht oder während Perioden geringer Sonnenintensität zu versorgen.

15. Vorrichtung nach Anspruch 14, wobei der Akkumulator (151) mit einem Leistungs-Resonator-Empfänger (152) verbunden ist, der imstande ist, in Benutzungsposition die von den Leistungs-Resonatoren-Empfängern (152) auf dem Boden des Vorratsbehälter (2, 200, 210) übertragene magnetische Energie in Elektrizität umzuwandeln.

16. Vorrichtung nach einem der Ansprüche 1 bis 15, wobei das untere Ende (112) des Mantels einen Reflektor (400, 401, 402, 403, 404, 501, 502) umfasst, der ausgebildet ist, um das Licht zum Mantel zu reflektieren.

17. Vorrichtung nach Anspruch 16, wobei der Reflektor (400, 401, 402, 403, 404) konvex ist.

18. Vorrichtung nach Anspruch 16, wobei der Reflektor (501, 502) konkav ist.

19. Vorrichtung nach einem der Ansprüche 16 bis 18, wobei der Reflektor eine Kontaktoberfläche (S1) mit dem unteren Ende des Mantels unter einer Oberfläche (S2) des unteren Endes des Mantels derart aufweist, dass ein Teil des Lichts durch das untere Ende des Mantels hindurchgelassen wird.

20. Vorrichtung nach einem der Ansprüche 16 bis 18, wobei der Reflektor (402) derart durchbrochen ist, dass ein Teil des Lichts zum unteren Ende des Mantels hindurchgelassen wird.

21. Vorrichtung nach einem der Ansprüche 1 bis 20, wobei die Glocke aus einem Material ist, das aus Polypropylen (PP), Poly(methylmethacrylat) (PMMA), Poly(ethylenterephthalat) (PET) und einem transparenten Kunststoff mit Anti-UV-Behandlung ausgewählt ist.

22. Vorrichtung nach einem der Ansprüche 1 bis 21, wobei der Mantel aus einem Material ist, das aus Polyester, Polypropylen (PP) und jedem anderen transparenten Folienmaterial mit einer Dicke von einigen Dutzend Mikrometern ausgewählt ist.

**23.** Vorrichtung nach einem der Ansprüche 1 bis 22, wobei der Mantel in Benutzung mit zuvor entgastem Salzwasser gefüllt ist.

**24.** Photo-Bioreaktor für die Herstellung einer konzentrierten Algenlösung, aufweisend einen Vorratsbehälter (2, 200, 210), der die Algenlösung enthält, **dadurch gekennzeichnet, dass** er mindestens eine Vorrichtung (100) zum Auffangen, Transportieren und Verbreiten von Licht nach einem der Ansprüche 1 bis 23 umfasst, die frei auf der Oberfläche der Algenlösung schwimmt.

**25.** Photo-Bioreaktor nach Anspruch 24, umfassend ferner Rührmittel der Algenlösung, die imstande sind, einen Strom zu erzeugen, der imstande ist, Vorrichtungen zum Auffangen, Transportieren und Verbreiten von Licht nach einem der Ansprüche 1 bis 24 frei schwimmend auf der Oberfläche der Lösung zu verlagern.

**26.** Photo-Bioreaktor nach Anspruch 25, wobei die Rührmittel einen Verteiler von Druckgas, insbesondere von Kohlendioxid, umfassen.

**27.** Photo-Bioreaktor nach einem der Ansprüche 13 bis 26, umfassend ferner eine Maschenstruktur (220), von der jede Masche (221) von einer Größe strikt größer als ein maximaler Durchmesser (D, D1, D2) der Glocke der mindestens einen Vorrichtung zum Transportieren und Verbreiten von Licht derart ist, dass eine begrenzte Zufallsbewegung einer Vorrichtung zum Transportieren und Verbreiten von Licht gestattet ist, die in einer Masche liegt.

**28.** Photo-Bioreaktor nach Anspruch 27, wobei die Maschenstruktur aus einem elastischen und schwimmenden Netz besteht.

**29.** Photo-Bioreaktor nach einem der Ansprüche 22 bis 28, umfassend ferner Leistungs-Resonatoren-Sender (152), die den Boden des Vorratsbehälters (2, 200, 210) ausstatten.

**30.** Vorrichtung zur Reinigung (300) einer Vorrichtung zum Auffangen, Transportieren und Verbreiten von Licht nach einem der Ansprüche 1 bis 23, **dadurch gekennzeichnet, dass** sie einen kreisförmigen Halter (301) umfasst, der mit mindestens einer rotierenden motorisierten Rolle (302) für den Rotationsantrieb der Glocke (120) der Vorrichtung (100) zum Auffangen, Transportieren und Verbreiten von Licht in Reinigungsposition ausgestattet ist, wobei der Halter ferner mindestens eine Bürste (303) umfasst, die unter dem Halter eingerichtet und bestimmt ist, mit dem Mantel (110) der Vorrichtung (100) zum Auffangen, Transportieren und Verbreiten von Licht in Reinigungsposition in Kontakt zu sein.

**31.** Reinigungsvorrichtung (300) nach Anspruch 30, wobei der Halter (301) ferner einen Schwimmer umfasst.

**32.** Photo-Bioreaktor nach einem der Ansprüche 24 bis 29, umfassend ferner mindestens eine Reinigungsvorrichtung nach einem der Ansprüche 30 oder 31.

**Claims**

**1.** Device (100) for capture, transport and diffusion of light in a solution (1) contained in a tank (2, 200, 210), the device comprising a cylindrical sleeve (110), made of material transparent to the transported and diffused light, the sleeve (110) being intended to be filled with a liquid suitable for transmitting and diffusing light and to balance the external pressure in order to keep the sleeve in cylindrical shape, **characterised in that** it further comprises a sealed bell (120), transparent to the captured, transported and diffused light, and formed at least partially by two walls (120a-120b) separated by a sealed interstice (e) partially or totally filled by gas, the bell comprising a domed portion (121) prolonged by a tubular portion (122), a first end (111) of the sleeve (110) being intended to be blocked against the bell (120) in such a way that in the position of use, the bell floats freely in the solution and keeps at least the domed portion (121) above the surface and the sleeve (110) filled with liquid below the surface and extended vertically under the bell by adopting a cylindrical shape.

**2.** Device according to claim 1, wherein the domed portion of the bell is hemispherical.

**3.** Device according to claim 1 or 2, wherein the domed portion of the bell is with a double wall.

**4.** Device according to claim 1 or 2, wherein the domed portion (603, 701a) of the bell is with a single wall and the tubular portion (602, 702) is with a double wall.

5. Device according to any of claims 1 to 4, wherein the sealed interstice (e) separating the double walls is filled exclusively with gas.

6. Device according to any of claims 1 to 4, wherein the sealed interstice (e2) separating the double walls is filled with an expanded foam (605, 702) with closed cells filled with gas, such as a polyurethane foam.

7. Device according to any of claims 1 to 6, wherein the domed portion comprises a tubular vent 130) passing through the domed portion in a sealed manner and comprising a valve (131) for maintaining atmospheric pressure provided with a sealed flexible membrane that can be deformed under atmospheric pressure.

8. Device according to any of claims 1 to 7, wherein the tubular portion of the bell comprises an annular groove (123) intended to receive, in use, an extensible O-ring (124) for keeping the seal of a first fastening end of the sleeve against the tubular portion.

9. Device according to any of claims 1 to 8 wherein the tubular portion of the bell has a conical surface (604a, 702a) tapering towards the domed portion.

10. Device according to claim 4, wherein the bell (600) comprises a first part (603) comprising the domed portion (601) prolonged by a cylindrical portion, and a second part (604) comprising a cylindrical portion (604a) provided with a neck (604b) intended to be in sealed peripheral contact with an inner surface (603a) of the first part, in the position of use, so as to delimit a sealed interstice (e2), in use, between the cylindrical portion of the first part and the cylindrical portion of the second part.

11. Device according to claim 4, wherein the bell (700) comprises a first part (701) comprising the domed portion (701a) prolonged by a cylindrical portion (701b), and a second part (702) comprising a cylindrical portion (702) provided with a shoulder (702c) intended to be in sealed peripheral contact with the cylindrical portion (701b) of the first part, in the position of use, the second part being formed by a ring made of material with closed cells filled with gas.

12. Device according to claim 10 or 11, wherein the cylindrical portion of the second part has a reflective surface (606, 706) to reflect, in use, the light coming from the domed portion towards the sleeve.

13. Device according to any of claims 1 to 12, wherein the sleeve comprises a second end provided with a ballasted sealed closure (112).

14. Device according to any of claims 1 to 13, wherein a lower end of the sleeve comprises photovoltaic cells (150) directed, in use, towards the bell, and connected to an accumulator (151) connected to light-emitting diodes (152) directed, in use, towards the bell, the accumulator being programmed to accumulate electricity during the day and to supply the light-emitting diodes at night or during periods of low solar intensity.

15. Device according to claim 14, wherein the accumulator (151) is connected to a power-emitting resonator (152) able to transform into electricity the magnetic energy transmitted, in the position of use, by power-emitting resonators (152) provided on the bottom of the tank (2, 200, 210).

16. Device according to any of claims 1 to 15, wherein the lower end (112) of the sleeve comprises a reflector (400, 401, 402, 403, 404, 501, 502) shaped to reflect the light towards the sleeve.

17. Device according to claim 16, wherein the reflector (400, 401, 402, 403, 404) is convex.

18. Device according to claim 16, wherein the reflector (501, 502) is concave.

19. Device according to any of claims 16 to 18, wherein the reflector has a contact surface (S1) with the lower end of the lower sleeve at a surface (S2) of said lower end of the sleeve so as to allow a portion of the light to pass through the lower end of the sleeve.

20. Device according to any of claims 16 to 18, wherein the reflector (402) is perforated so as to allow a portion of the light to pass towards the lower end of the sleeve.

21. Device according to any of claims 1 to 20, wherein the bell is made from a material chosen from polypropylene (PP), poly(methyl methacrylate) (PMMA), poly(ethylene terephthalate) (PET), and a UV-treated transparent plastic.

22. Device according to any of claims 1 to 21, wherein the sleeve is made from a material chosen from polyester, polypropylene (PP) and any other transparent materials made of film, of a thickness of few tens of micrometres.

23. Device according to any of claims 1 to 22, wherein the sleeve, in use, is filled with salt water and degassed beforehand.

24. Photo-bioreactor for the production of a concentrated algal solution comprising a tank (2, 200, 210) containing the algal solution, **characterised in that** it comprises at least one device (100) for capture, transport and diffusion of light according to any of claims 1 to 23 freely floating on the surface of the algal solution.

25. Photo-bioreactor according to claim 24, further comprising means for stirring the algal solution able to generate a current that can displace devices for capture, transport and diffusion of light according to any of claims 1 to 24 freely floating on the surface of the solution.

26. Photo-bioreactor according to claim 25, wherein the means for stirring comprise a pressurised gas distributor, in particular carbon dioxide.

27. Photo-bioreactor according to any of claims 13 to 26, further comprising a meshed structure (220) of which each mesh (221) is of a dimension strictly greater than a maximum diameter (D, D1, D2) of the bell of said at least one device for the transport and diffusion of light, so as to allow for a delimited random movement of a device for transport and diffusion of light comprised in a mesh.

28. Photo-bioreactor according to claim 27, wherein the meshed structure is formed by a flexible and floating net.

29. Photo-bioreactor according to any of claims 22 to 28, further comprising power-emitting resonators (152) provided on the bottom of the tank (2, 200, 210).

30. Device for cleaning (300) a device for capture, transport and diffusion of light according to any of claims 1 to 23, **characterised in that** it comprises a circular support (301) provided with at least one rotating motorised roller (302) for the driving in rotation of the bell (120) of the device (100) for capture, transport and diffusion of light in the cleaning position, the support further comprising at least one brush (303) arranged under the support and intended to be in contact with the sleeve (110) of the device (100) for capture, transport and diffusion of light in cleaning position.

31. Device for cleaning (300) according to claim 30, wherein the support (301) further comprises floaters.

32. Photo-bioreactor according to any of claims 24 to 29, further comprising at least one device for cleaning according to any of claims 30 or 31.

Fig. 1

Fig. 2

Fig. 3

Fig. 4

Fig. 5

Fig. 6

Fig. 7

Fig. 8

200

100

221

220

210

100

121

120

100

2

122

1

123

124

111

110

Fig. 9

150

152

152

151

Fig. 10

100

152

152

153

151

154

120

100

302

300

302

303

Fig. 11

303

110

302

300

100

120

302

F6

301

Fig. 12

Fig. 13

Fig. 14

Fig. 15

Fig. 16

Fig. 17

Fig. 18

RL1

L1

L2

501

501

Fig. 19

502

Fig. 20

603

Fig. 21

603a

F7

604b

604

604a

Fig. 22

601

600

602

e2

604d

605

604c

606

F8

F8

701a

701

F9

Fig. 23

701b

706

702c

702

702b

702a

Fig. 24

701

700

702

706

**EP 3 647 406 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2013011448 A
- WO 2016087779 A